(19)

Europäisches
Patentamt
European
Patent Office
Office européen
des brevets

(11) **EP 3 095 483 A1**

(12) **EUROPEAN PATENT APPLICATION**
published in accordance with Art. 153(4) EPC

(43) Date of publication:
**23.11.2016 Bulletin 2016/47**

(21) Application number: **14874083.0**

(22) Date of filing: **24.12.2014**

(51) Int Cl.:
***A61N 5/10*** *(2006.01)*          ***A61L 31/00*** *(2006.01)*

(86) International application number:
**PCT/JP2014/084029**

(87) International publication number:
**WO 2015/098904 (02.07.2015 Gazette 2015/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **25.12.2013 JP 2013266214**

(71) Applicants:
• **National University Corporation Kobe University
Kobe-shi, Hyogo 657-8501 (JP)**
• **Alfresa Pharma Corporation
Osaka-shi, Osaka 540-8575 (JP)**
• **Kanai Juyo Kogyo Co., Ltd.
Itami-shi, Hiyogo 664-0025 (JP)**

(72) Inventors:
• **FUKUMOTO, Takumi
Kobe-shi
Hyogo 657-8501 (JP)**
• **SASAKI, Ryohei
Kobe-shi
Hyogo 657-8501 (JP)**

• **MORIHARA, Tsutomu
Noda-shi
Chiba 270-0216 (JP)**
• **SASAI, Yoshio
Tokyo 103-0023 (JP)**
• **FURUTA, Yoshihisa
Noda-shi
Chiba 270-0216 (JP)**
• **OBATA, Tsutomu
Itami-shi
Hyogo 664-0025 (JP)**
• **TAGAMI, Yoshitaka
Itami-shi
Hyogo 664-0025 (JP)**

(74) Representative: **Isarpatent
Patent- und Rechtsanwälte Behnisch Barth
Charles
Hassa Peckmann & Partner mbB
Friedrichstrasse 31
80801 München (DE)**

(54) **RADIOTHERAPY SPACER**

(57)    A radiotherapy spacer is disclosed that has both flexibility for allowing it to gently cover the organs of various shapes along their surfaces and pressure resistance for preventing itself from being crushed under the pressure between organs and thus enabling it to retain its own shape and continuously ensure the therapeutic space. The spacer comprises a fiber assembly formed of entangled biocompatible fibers, which may be bioabsorbable, such as nonwoven fabric, and is characterized in that the density of the fiber assembly is 0.05 g/cm$^3$ to 0.2 g/cm$^3$.

EP 3 095 483 A1

**Description**

TECHNICAL FIELD

The present invention relates to a radiotherapy spacer.

BACKGROUND ART

Cancer is the leading cause of death of Japanese people, and more than a half of those who die of cancer are aged people over 75 year old. Since it is difficult in many cases to perform substantially invasive, radical surgery on an aged person for the treatment of such cancers as pancreatic, liver, and bile duct cancers, development of a radical cure is desired which is less invasive yet as effective as surgery.

An example of low invasive treatment of cancer is a radiotherapy including particle beam therapy and implant radiation, but considering radiation damage to the normal tissue, even radiotherapy faces a difficulty in irradiating a tumor with a radiation dosage necessary for radical treatment when a normal tissue is present around it. Thus a method for treatment, a combination of surgery and radiotherapy, is becoming popular, in which a space is surgically ensured between the cancer and surrounding normal tissues, and while protecting the normal tissues around the cancer from exposure to radiation by that therapeutic space, the tumor is irradiated with a sufficient radiation dosage necessary for radical treatment. In this type of therapy, a medical device used for keeping a therapeutic space between the cancer and normal tissues is called a "spacer".

PRIOR ART DOCUMENTS

PATENT DOCUMENTS

[Patent Document 1] WO2011/055670

SUMMARY OF INVENTION

TECHNICAL PROBLEM

At present, a spacer for radiotherapy including particle beam therapy has not yet been on the market either in Japan or out of the country. Thus, conventional medical devices such as an artificial blood vessel, pericardium sheet, tissue expander (silicone bag), collagen sponge, and the like are employed as alternatives to a pacer. However, such alternative medical devices are not absorbable by the body, and they increase risks of reoperation and infection. Further, as they are note intended to be used as spacers in the first place, they entail such inconveniences that they have poor ability of shielding radiation, and that they are not easy to process but are costly.

Aiming to solve these problems, a radiotherapy spacer has been proposed which comprises a fiber assembly formed of three-dimensionally entangled fibers of bioabsorbable synthetic polymers (Patent Document 1). This radiotherapy spacer exhibits excellent effects in that it offers protection of surrounding normal tissues by efficaciously shielding them from radiation utilizing the water which the fiber assembly retains, and also in that reoperation becomes no longer necessary for removing it because it is made of a bioabsorbable material.

From a surgical point of view, it is essential for a spacer that is to be implanted in the body to have such properties that allow itself to gently fit organs along their surfaces, while keeping its own shape thus brought about. Thus, a spacer must have not only such flexibility that allows itself to gently cover organs of various shapes but also certain pressure resistance so that it can prevent itself from being crushed, thus retain its own shape and continuously ensure the therapeutic space, even under the pressure between organs. However, if flexibility is required with the spacer described in Patent Document 1, thinner fibers must be employed, which will lower its pressure resistance. On the other hand, if increased pressure resistance is sought, thicker fibers must be employed, which will reduce its flexibility. Patent Document 1 is silent about how to achieve such flexibility and pressure resistance simultaneously. Namely, there has been provided no radiotherapy spacer that has, in proper balance, both flexibility for allowing itself to gently cover organs of various shapes and pressure resistance necessary to prevent itself from being crushed, thus retain its own shape and continuously ensure the therapeutic space, even under the pressure between organs. Further, with the spacer described in Patent Document 1, if its voids are crushed, the level of its water content is lowered, making its radiation shielding efficacy insufficient.

SOLUTION TO PROBLEM

As a result of intense studies, the present inventors found that the problem noted above can be solved by achieving a proper balance between the flexibility and pressure resistance of a spacer, by adjusting the density of the fiber assembly.

Thus, the invention made to solve the above-mentioned problem is as follows:

A radiotherapy spacer comprising a fiber assembly of entangled biocompatible fibers, wherein the density of the fiber assembly is 0.05 g/cm$^3$ to 0.2 g/cm$^3$.

With this radiotherapy spacer, by adjusting its density to 0.05 g/cm$^3$ to 0.2 g/cm$^3$, a proper balance between its voids and fibers can be achieved so as to realize both flexibility and pressure resistance of the fiber assembly in proper balance. As a result, the radiotherapy spacer exhibits flexibility without employing thinner fibers for producing the fiber assembly, and pressure resistance without employing thicker fibers. Hence, the radiotherapy spacer can possess both flexibility for allowing itself to gently cover organs of various shapes along their surfaces and pressure resistance for preventing itself from being crushed and thus enabling it to retain its own shape and continuously keep the therapeutic space, even under the pressure between organs.

The fiber assembly mentioned above may preferably be nonwoven fabric. By employing nonwoven fabric as a fiber assembly, a radiotherapy spacer is provided consisting of randomly and three-dimensionally entangled fibers, and holding voids of various sizes within it, and whose density and thickness can be easily adjusted, and able to efficiently and effectively achieve both flexibility and pressure resistance at the same time.

The above biocompatible fibers may preferably be bioabsorbable. By employing bioabsorbable fibers as the fiber assembly-forming biocompatible fibers, the resulting radiotherapy spacer will, when implanted in a cancer patient, gradually be absorbed by the body and will disappear after completion of the radiotherapy, eliminating therefore the need of reoperation for removing the implanted spacer and improving the QOL (Quality of Life) of the cancer patient.

The thickness of the fiber assembly may preferably be 5 mm to 15 mm. By adjusting the thickness of the fiber assembly within this range, the resulting radiotherapy spacer can possess both the above-mentioned flexibility and pressure resistance, ensure a therapeutic space between the tumor and surrounding normal tissues, and protect the normal tissues around the tumor from radiation exposure without hindering irradiation of the tumor with a radiation dosage required for radical treatment, thereby achieving the essential functions of as spacer.

(Definitions)

The term "Spacer therapy" means a method of treatment in which a therapeutic space is ensured between a tumor and surrounding normal tissues, and while protecting, with the treatment space, the normal tissues around the tumor from exposure to radiation, the tumor is irradiated with a sufficient radiation dosage necessary for radical treatment. The term "spacer" means a medical device used in spacer treatment to ensure a space between the tumor and surrounding normal tissues. The term "biocompatible" refers to having a property of fitting organism without exerting influence on nor receiving influence from it.

EFFECTS OF INVENTION

As explained above, as the density of the fiber assembly is 0.05 g/cm$^3$ to 0.2 g/cm$^3$, the radiotherapy spacer according to the present invention can ensure a therapeutic space between a tumor and surrounding normal tissues, thereby realize the required performance as a spacer, i.e., protection of surrounding normal tissues by providing efficient shielding with water held in the fiber assembly, and further simultaneously achieve, in proper balance, both flexibility for allowing it to gently cover the organs of various shapes along their surfaces and pressure resistance for enabling it to retain its own shape without being crushed and continuously ensure the therapeutic space under the pressure between organs, and therefore is applicable to all the radiotherapies including particle beam therapy and implant radiation.

DESCRIPTION OF EMBODIMENTS

Embodiments of the present invention are described in detail below. The present invention, however, is not limited to those embodiments.

1. Radiotherapy spacer

The radiotherapy spacer comprises a fiber assembly of entangled biocompatible fibers. Because throughout this fiber assembly which is formed of three-dimensionally entangled biocompatible fibers, there are defined countless communicating voids between fibers, and those voids can work as a radiation shield when retaining water therein and at same time give the radiotherapy spacer its lightness and flexibility as a whole.

The density of the fiber assembly that forms the radiotherapy spacer is 0.05 g/cm$^3$ to 0.2 g/cm$^3$. As the density of the fiber assembly is adjusted within this range, the radiotherapy spacer can simultaneously achieve, in proper balance, both flexibility for allowing itself to gently cover the surface of organs of various shapes and pressure resistance for enabling it to retain its own shape without being crushed under the pressure between organs and continuously ensure the therapeutic space. In more detail, because the density of the fiber assembly that forms the radiotherapy spacer is adjusted within the above range, the countless communicating voids defined in the fiber assembly are arranged therethrough so as to give, in the best balance, both flexibility and pressure resistance simultaneously. As a result, it is possible to attain flexibility without reducing the thickness of the fibers forming the fiber assembly, while achieving pressure resistance without increasing the thickness of the fibers. Further, when the density of the fiber assembly forming the radiotherapy spacer falls within the above range, the required performance of the spacer can be fully achieved, i.e., ensuring a therapeutic space between a tumor and surrounding normal tissues and thus providing an efficient radiation shield with the water held in the fiber assembly to protect surrounding tissues.

Besides, if the aforementioned density is less than 0.05 g/cm$^3$, the spacer for radiotherapy is likely to be crushed after implanted in the body due to the pressure between organs, and thus become less able to keep its own shape to ensure the therapeutic space, thereby failing to maintain a sufficient amount of water deeded to offer a radiation shield. Further, if the aforementioned density exceeds 0.2 g/cm$^3$, the radiotherapy spacer will have reduced flexibility and become unable to gently fit the surface of organs of various shapes after implanted in the body, thus failing to effectively perform a spacer therapy.

The structure of the fiber assembly that forms the radiotherapy spacer must hold countless communicating voids between fibers so that the fiber assembly can retain water in it in order to provide a radiation shield, and may, for example, be a three-dimensional fabric, three-dimensional knit, or nonwoven fabric.

Among them, the fiber assembly is preferably a nonwoven fabric. By adopting nonwoven fabric as the structure of the fiber assembly, the radiotherapy spacer is provided as consisting of fibers that are randomly and three-dimensionally entangled, with communicating voids of various sizes defined in the fiber assembly, which allows easy adjustment of its density and thickness. As a result, flexibility and pressure resistance can both be efficiently and effectively achieved in proper balance in the radiotherapy spacer. Further, if it is nonwoven fabric-based, the radiotherapy spacer can be produced at a reduced cost, for nonwoven fabric allows production of a bulky fiber assembly using a relatively small amount of fibers.

The fiber assembly forming the radiotherapy spacer is made of entangled biocompatible fibers. Such biocompatible fibers may be employed that are permitted as medical devices, are nontoxic to the body or cells, and free of infection risks.

The above biocompatible fibers may properly be bioabsorbable. If the biocompatible fibers employed are bioabsorbable, a radiotherapy spacer thus obtained will begin to be subject to gradual decomposition and absorption by the body after implanted in a cancer patient during a spacer treatment, and entirely disappear from the body following the completion of radiotherapy. Thus, by employing bioabsorbable fibers as biocompatible ones, no reoperation to remove the implanted spacer becomes necessary, which can not only greatly reduce the risk of infection but improve the QOL of cancer patients.

Though there is no particular limitation as to the type of the above bioabsorbable, biocompatible fibers, they are preferably not of animal origin but chemically synthesized polymers, which are less likely to provoke inflammatory, immune, or thrombus forming responses in the tissues or cells of the body. Examples of such bioabsorbable polymers include, but not limited to, poly(ester-ether)s, poly(ester carbonate)s, poly(acid anhydride)s, polycarbonate, poly(amide-ester)s, polyacrylates, and inorganic polymers. More specifically, at least one compound can be selected from the group consisting of poly(glycolic acid), poly(L-lactic acid), poly(DL-lactic acid), polyglactin (D/L=9/1), polydioxanone, glycolide/trimethylene carbonate (9/1), polycaprolactone, lactides (D, L, DL forms), glycolide-lactide (D, L, DL forms) copolymers, glycolide-ε-caprolactone copolymers, lactide (D, L, DL forms)-ε-caprolactone copolymers, poly(p-dioxanone), and glycolide-lactide (D, L, DL forms)-ε-caprolactone copolymers.

The biocompatible fibers mentioned above may also be a non-bioabsorbable synthetic polymer. A non-bioabsorbable synthetic polymer may be at least one selected, for example, from polyester, polyethylene, polypropylene, polybutester, polytetrafluoroethylene, polyamide, polyvinylidene fluoride, polyurethane, and vinylidene fluoride-hexafluoropropylene.

The thickness of the fiber assembly forming a radiotherapy spacer may preferably be 5 mm to 15 mm. By adjusting the thickness of the fiber assembly within this range, the radiotherapy spacer according to the present invention can hold a sufficient amount of water to provide a shield necessary for a spacer therapy and ensure a sufficient therapeutic space to protect surrounding normal tissues from radiation, by simultaneously achieving, in proper balance, both flexibility necessary for allowing itself to gently cover organs of various shapes along their surfaces and pressure resistance for enabling it to retain its own shape without being crushed and continuously ensure the therapeutic space under the pressure between organs. If the thickness of the fiber assembly is less than 5 mm, it may be difficult for the radiotherapy spacer to hold a sufficient amount of water, which may result in a reduced radiation shielding effect. Further, if the thickness is greater than 15 mm, the radiotherapy spacer may overly expand a therapeutic space when implanted in the body of a cancer patient, which may press surrounding organs and thus impose excessive burdens on the body. Besides, it is also possible to overlay two or more spacers in order to adjust the total thickness of the fiber assemblies to 5 mm to 15 mm.

2. Method for production of a radiotherapy spacer

The method for production of a radiotherapy spacer is described below. Whether the fiber assembly to be used to form a radiotherapy spacer is three-dimensional fabric, three-dimensional knit, or nonwoven fabric in structure, any of the fiber assembly can be produced by conventional means. Where the fiber assembly is of nonwoven fabric in structure, the radiotherapy assembly may be produced by such a processing as needle punching, chemical bonding, thermal bonding, spunlacing, or the like.

Considering their use in cancer patients, the process of production of the radiotherapy spacers may preferably involve a sterilization step. The sterilization step may be realized by producing spacers in an aseptic room or by sterilizing spacers after there are formed. Examples of the method for sterilization include autoclave sterilization, EOG sterilization, gamma irradiation sterilization, electron beam sterilization, plasma sterilization, and the like.

3. Way of use of a radiotherapy spacer

The way of use of the radiotherapy spacer is described below. The radiotherapy spacer, sterilized, is surgically placed in a cancer patient, around the tumor to be treated, and implanted so as to create a therapeutic space between the tumor and surrounding organs. For this surgery for implantation, the density, thickness, size, and shape of the radiotherapy spacer may have been adjusted as desired in accordance with the type of the cancer or the position of the body where it is to be implanted. Further, two or more radiotherapy spacers may be used on top of each other.

The fiber assembly may contain water when the radiotherapy spacer is implanted in the body. In that case, there is no specific limitation as to the rate of water content of the radiotherapy spacer insofar as it retains such an amount of water that offers a sufficient shield against radiation.

After implantation of the radiotherapy spacer in the body, radiotherapy is started. In this type of radiotherapy, the radiotherapy spacer maintains, in good balance, both of its flexibility necessary for allowing itself to gently cover the surrounding organs in the body of the cancer patient and its pressure resistance for enabling it to retain its own shape without being crushed and continuously ensure the therapeutic space under the pressure between organs, and thus enables, by means of the therapeutic space, protection of normal tissues around the tumor from radiation while not hindering irradiation of the target tumor with a radiation dosage for radical treatment. After completion of the radiotherapy, since the radiotherapy spacer comprising bioabsorbable, biocompatible fibers will be absorbed by the body and disappear, no reoperation will be needed for its removal and also a risk of infection thus will be reduced. In this spacer therapy, irradiation with a radiation dosage for radical treatment will be performed in all the type of radiation therapies including X ray therapy; particle beam therapies such as proton beam therapy, carbon ion beam therapy; implant therapies using iridium; iodide and the like.

The types of cancers to be treated include, for example, head and neck cancer, skull base neoplasm, non-small-cell lung cancer, mediastinal tumor, hepatocellular carcinoma, pancreatic cancer, stomach cancer, prostate cancer, colorectal cancer, vaginal cancer, metastatic tumor (monostotic), musculoskeletal tumors, and the like.

The radiotherapy spacer according to the present invention is not limited to the embodiments mentioned above. For example, the radiotherapy spacer according to the present invention may be provided, in the surface layer of the fiber assembly, with a material to prevent adhesion with the body. By thus providing a material for preventing adhesion in the surface layer, the radiotherapy spacer according to the present invention enables prevention of adhesion between the fiber assembly and surrounding tissues after surgical implantation of the spacer, and reduce the risks of complications in the cancer patient, thereby making it possible to conduct spacer therapy on the patient with increased efficacy. Such materials for prevention of adhesion may, for example, be what is to be coated on the surface of the fiber assembly, or a film-like member as a covering membrane.

[Examples]

The present invention is described in further detail below. The present invention, however, is not limited to the examples below.

In order to validate the flexibility and pressure resistance of the radiotherapy spacer in the body, physical tests were carried out assuming when it is implanted in a human body

<Spacer>

Using polyglycolic acid fibers, which is a bioabsorbable synthetic polymer, as the raw material, spacers to be used in the test (hereinafter referred to also as "test spacer") were produced in the form of a nonwoven fabric by a conventional method. More specifically, the material polyglycolic acid fibers were cut into short fibers, which then were processed into a web, a sheet of fiver, and then processed by needle punching into a sheet of unwoven fabric. The test spacer produced in this manner is in the shape of a plate which generally is a rectangular parallelepiped. The density of the test spacers were adjusted in the processing of the unwoven fabric. The respective test spacers of Examples 1-3 and Comparative example 1 described later were made of the same fibers, and their shape and dimensions were identical, while they differed in their density.

[Example 1]

Test spacers with a density adjusted to 0.05 $g/cm^3$.

[Example 2]

Test spacers with a density adjusted to 0.1 $g/cm^3$.

[Example 3]

Test spacers with a density adjusted to 0.2 $g/cm^3$.

[Comparative Example 1]

Test spacers with a density adjusted to 0.3 $g/cm^3$.

<Methods>

When a test spacer is implanted in the body of a human, the plate-like surfaces of the test spacer will receive a load from organs. The flexibility and pressure resistance that the test spacers will exhibit when they are implanted in a human body were determined.

(A) Flexibility

The flexibility of the test spacer was determined as its flexural property observed when a pressure was applied to a certain area of the plate-like surface of the spacer. Specifically, three-point bending test was performed to measure its flexural property in accordance with JIS K 7171 (Test method for flexural property - plastics). Five samples of each test spacer of Examples 1-3 and Comparative Example 1, which were cut into 10 mm in length, 100 mm in width, and 5 mm in thickness, were prepared, each of which was attached to the testing apparatus. Measurement was carried out under the following conditions: the initial position of the loading pin: 6 mm above the test spacer, maximum sinking: 30 mm below the test spacer, test speed: 100 mm/sec, temperature: 20 °C, humidity: 65% RH, and the maximum flexural stress then was calculated for each test spacer. In the measurement, the value of the first measurement was not counted, but the maximum and the minimum values were recorded on the second to the sixth measurements (5 times in total), and the mean value of these five measurements was calculated. The area to which the pressure was applied was 1.57 $cm^2$. It was observed by naked eye in the three-point bending test that those test spacers had the optimum level of flexibility that was suitable for their gently fitting organs if their maximum flexural stress was not higher than 350 kPa. Thus, it was determined that the test spacer would exhibit the optimum flexibility suitable for its gently fitting organs if its maximum flexural stress was not higher than 350 kPa.

(Pressure resistance)

The pressure resistance of the test spacers was determined as the rate of the remaining thickness after a pressure in the direction of the thickness was applied to nearly the entire surface of the spacers' plate-like surface. Three samples of each test spacer of Examples 1-3 and Comparative Example 1, which were cut into 63.5 mm in width, 40 mm in length, and 5 mm in thickness, were prepared, each of which was attached to the testing apparatus. The nearly entire area to which the pressure was applied was approximately 20.5 cm$^2$. In accordance with JIS L 1912:1997 (test method of nonwoven fabric for medical use), assuming the thickness under the pressure of 0.5 kPa as the initial thickness under the initial load, the thickness under an increased load was measured, the mean value of three samples determined, and the rate (%) of remaining thickness was calculated as shown in Equation 1 below.

[Math 1]

$$\text{Rate of remaining thickness (\%)} = \frac{\text{thickness under load (mm)}}{\text{thickness (mm) under load of 0.5 kPa}} \times 100\% \quad \cdots (1)$$

Supposing, in the above, spacer treatment of pancreatic cancer, the test spacer implanted in the body would receive on its plate-like surface a load mainly of organs such as the liver, intestines, and the like. The pressure "P" applied by the average weight of 1.4 kg of the liver, the heaviest organ in the human body, to the area of the plate-like spacer surface of 100 cm$^2$ (10 cm x 10 cm) is calculated as 1.37 kPa as shown in equation 2 below.

[Math 2]

$$P = \frac{1.4 \text{ (kgf)} \times 9.80665 \text{ (N/kgf)}}{0.1 \times 0.1 \text{ (m}^2)} = 1.37 \text{ (kPa)} \quad \cdots\cdots\cdots (2)$$

The pressure that the test spacer would receive from organs when implanted in the body was estimated as 1.5 kPa, i.e., the sum of the above 1.37 kPa and a load of intestines and the like. If the rate of remaining thickness was not less than 85% under this pressure of 1.5 kPa was applied to the plate-like surface of the test spacer, it was judged that the test spacer would exhibit an optimum pressure resistance, without being crushed under the pressure between organs, and maintain its own shape and ensure the therapeutic space.

The results of the test of flexibility and pressure resistance are shown in Table 1 below.

[Table 1]

| | Density (g/cm$^3$) | Flexibility | Pressure resistance | | |
| --- | --- | --- | --- | --- | --- |
| | | Maximum flexural stress (kPa) | Thickness (mm) | | Remaining rate (%) |
| | | | 0.5 kPa loaded | 1.5 kPa loaded | |
| Example 1 | 0.05 | 26.12 | 4.96 | 4.43 | 89 |
| Example 2 | 0.1 | 31.82 | 6.6 | 6.21 | 94 |
| Example 3 | 0.2 | 234.23 | 6.43 | 6.1 | 95 |
| Comparative Example 1 | 0.3 | 794.03 | 6.79 | 6.61 | 97 |

<Assessment>

As seen in Table 1, each of Examples 1-3 and Comparative Example 1 showed a rate of remaining thickness that is greater than 85%, suggesting that they had an optimum pressure resistance that would prevent them from being crushed, and thus enable them to retain their shape and continuously ensure the therapeutic space. Simultaneously, the maximum flexural stress observed with Examples 1-3 was not greater than 350 kPa, indicating that they had an optimum flexibility suitable for their gently fitting the surface of organs, whereas the maximum flexural stress observed with Comparative Example 1 was a value much greater than 350 kPa. Thus, it was confirmed that Examples 1-3, in comparison with Comparative Example 1, exhibited both an optimum flexibility that would allow them to gently fit the surface of organs and an optimum pressure resistance that would prevent those spacers from being crushed by pressure between organs, and thus retain their own shape and thus continuously ensure the therapeutic space.

In addition to Examples 1-3 and Comparative Example 1 mentioned above, another test, under a condition closer to the environment inside the body, was carried out to further confirm that the radiotherapy spacer according to the present invention had a sufficient flexibility and pressure resistance.

<Spacer>

The spacers used in the additional test (hereinafter referred also to "additional test spacers") were produced in the same manner as in Examples 1-3 and Comparative Example 1.

[Example 4]

Additional test spacers with a density adjusted to 0.05 g/cm$^3$.

[Example 5]

Additional test spacers with a density adjusted to 0.1 g/cm$^3$.

[Example 6]

Additional test spacers with a density adjusted to 0.2 g/cm$^3$.

[Comparative Example 2]

Additional test spacers with a density adjusted to 0.03 g/cm$^3$.

[Comparative Example 3]

Additional test spacers with a density adjusted to 0.25 g/cm$^3$.

(AA) Additional test of flexibility (water contained)

The flexibility of the additional test spacers was determined as their flexural property observed when a pressure was applied to a certain area of the plate-like surface of the spacers that were fully impregnated with water. Specifically, three-point bending test was performed to measure its flexural property in accordance with JIS K 7171 (Test method for flexural property - plastics). Two samples of each additional test spacer of Examples 4-6 and Comparative Examples 2-3, which were cut into 10 mm in length, 100 mm in width, and 5 mm in thickness, were prepared. Each sample, which was fully impregnated with water, was attached to the testing apparatus. Measurement was carried out under the following conditions: the initial position of the loading pin: 6 mm above the additional test spacer, maximum sinking: 30 mm below the additional test spacer, test speed: 100 mm/sec, temperature: 20 °C, humidity: 65% RH; and the maximum flexural stress then was calculated for each additional test spacer. In the measurement, the first of the measurements was not counted, but the maximum and the minimum values were recorded on the second to the sixth measurements (5 times in total), and the mean value of these five measurements was calculated. The area to which the pressure was applied was 1.57 cm$^2$. It was observed by naked eye that those additional test spacers had the optimum level of flexibility that was suitable for their gently fitting organs if their maximum flexural stress was not higher than 350 kPa in the three-point bending test, as in the result of the test (A) Flexibility. Thus, it was concluded that the additional test spacer would exhibit an optimum flexibility for allowing itself to gently fit organs if its maximum flexural stress was not higher than 350 kPa.

(BB) Additional test of pressure resistance (implanted in rats)

The pressure resistance of the additional test spacers was determined as the rate of remaining thickness by means of their implantation test in the abdominal cavity of rats as follows. Namely, three samples of each additional test spacer of Examples 4-6 and Comparative Examples 2-3, which were cut into approximately 30 mm in length, approximately 40 mm in width, and approximately 4-6 mm in thickness, were prepared and then sterilized with ethylene oxide gas. CD(SD)-strain male rats, eight-week old, were provided, and sterilized dry additional test spacers of Examples 4-6 and Comparative Examples 2-3 were implanted in the middle of the rats' abdomen under general anesthesia, Twenty-four hours after implantation, the rats' abdomen was incised, the additional test spacers of Examples 4-6 and Comparative Examples 2-3 were taken out and measured for their thickness. The rate (%) of remaining thickness was calculated by dividing the thickness of the additional test spacer after implantation by the thickness of the additional test spacer before implantation. In accordance with the above test (B) Pressure resistance, it was judged that the additional test spacer, if its rate of remaining thickness was not less than 85%, would exhibit an optimum pressure resistance in the body, without being crushed under the pressure between organs, and maintain its own shape and ensure the therapeutic space, and also that the pressure resistance was regarded to be sufficient from the clinical point of view if the rate of remaining thickness was not less than 60% in the body.

<Results>

The results of the additional test of flexibility and pressure resistance are shown in Table 2.

[Table 2]

| | Density (g/cm$^3$) | Flexibility | | | Pressure resistance | | |
|---|---|---|---|---|---|---|---|
| | | Thickness (mm) | | Maximum fractural stress, impregnate with water (kPa) | Thickness (mm) | | Remaining rate (%) |
| | | Dry | Impregnated with water | | Before implantation | After implantation | |
| Comparative Example 2 | 0.03 | 5 | 1.71 | 61.95 | 5.03 | 1.57 | 31% |
| Example 4 | 0.05 | 5 | 3.06 | 31.09 | 4.74 | 3.04 | 64% |
| Example 5 | 0.1 | 5 | 4.89 | 40.22 | 5.14 | 4.58 | 89% |
| Example 6 | 0.2 | 5 | 5.19 | 317.11 | 5.28 | 5.24 | 99% |
| Comparative Example 3 | 0.25 | 5 | 5.07 | 594.34 | 5.40 | 5.35 | 99% |

<Assessment>

1. Flexibility (impregnated with water)

Table 2 shows that the maximum flexural stress with any of Comparative Example 2 and Examples 4-6 was less than 350 kPa, indicating they would exhibit an optimum flexibility allowing them to gently fit organs. On the contrary, with Comparative Example 3, the maximum flexural stress was found far exceeding 350 kPa (approximately 594 kPa), indicating that it lacked flexibility and was not suitable for use.
Of the additional test spacers, Comparative Example 2 and Examples 4-5 showed reduction in their thickness after impregnated with water. It is considered that this was because when a spacer with relatively low density is impregnated with water, the water coming between fibers draws them closer to one another by its tensile force and causes, as a result, the spacer to shrink in thickness. With Comparative Example 2, whose density is particularly low compared with Examples 4-6 and Comparative Example 3, the thickness greatly reduced after impregnation with water, i.e., to 1.71 mm, about a third of the thickness presented before impregnation with water, which makes it incapable of functioning sufficiently as a spacer for providing a gap between organs. In contrast, Examples 4-6 can adequately function as a spacer. These results thus indicates that Examples 4-6, compared with Comparative Examples 2-3, function as a spacer for providing a gap between organs while simultaneously exhibiting an optimum flexibility allowing them to gently fit organs.

2. Pressure resistance (implanted in rats)

Table 2 shows that with Examples 5-6 and Comparative Example 3, the rate of remaining thickness after implanted in a rat's body exceeded 85% in every case, exhibiting an optimum pressure resistance that prevents those spacers from being crushed by pressure between organs and thus enable them to retain their own shape and continuously ensure the therapeutic space. Further, the rate of remaining thickness (64%) was lower than 85% with Example 4 after implantation. It was considered that this was because a spacer with a low density is more likely to be crushed under the influence of the body fluid and pressure between organs in a rat body. From the clinical viewpoint, however, if the rate of remaining thickness of a spacer is not less than 60% in the body, the spacer can ensure a therapeutic space. Thus, Example 4 has a sufficient pressure resistance that prevents the spacer from being crushed by the pressure between organs and thus enables it to retain its own shape and ensure a therapeutic space. On the other hand, with Comparative Example 2, the rate of remaining thickness was very low, i.e., 31%, indicating that Comparative Example 2 is incapable of functioning sufficiently as a spacer for providing a gap between organs. It is considered that the body fluid and the pressure between organs after implantation caused Comparative Example 2, the spacer with the lowest density, to crush from its pre-implantation dry state.

3. Flexibility and pressure resistance

By the results of the additional test described above, it was confirmed that Examples 4-6, in comparison with Comparative Examples 2 and 3, exhibit both an optimum flexibility allowing them to gently fit organs along their surfaces and an optimum pressure resistance that prevents those spacers from being crushed by pressure between organs and thus enables them to retain their own shape, thus continuously ensuring the therapeutic space, simultaneously.

[Industrial Applicability]

As mentioned above, the present invention can be utilized in radiotherapies such as X ray therapy, gamma ray therapy, proton beam therapy, and heavy particle therapy.

**Claims**

1. A radiotherapy spacer comprising a fiber assembly of entangled biocompatible fibers, wherein the density of the fiber assembly is 0.05 g/cm$^3$ to 0.2 g/cm$^3$.

2. The radiotherapy spacer according to claim 1, wherein the fiber assembly is a nonwoven fabric.

3. The radiotherapy spacer according to claim 1 or 2, wherein the biocompatible fibers are bioabsorbable.

4. The radiotherapy spacer according to one of claims 1-3, wherein the thickness of the fiber assembly is 5 mm to 15 mm.

## INTERNATIONAL SEARCH REPORT

| International application No. |
|---|
| PCT/JP2014/084029 |

A. CLASSIFICATION OF SUBJECT MATTER
*A61N5/10*(2006.01)i, *A61L31/00*(2006.01)i

According to International Patent Classification (IPC) or to both national classification and IPC

B. FIELDS SEARCHED

Minimum documentation searched (classification system followed by classification symbols)
A61N5/10, A61L31/00

Documentation searched other than minimum documentation to the extent that such documents are included in the fields searched
Jitsuyo Shinan Koho         1922–1996    Jitsuyo Shinan Toroku Koho    1996–2015
Kokai Jitsuyo Shinan Koho   1971–2015    Toroku Jitsuyo Shinan Koho    1994–2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2011/055670 A1  (Kobe University),<br>12 May 2011 (12.05.2011),<br>paragraph [0031]; fig. 1 to 3<br>& JP 2014-64940 A       & US 2012/0271093 A1<br>& EP 2497534 A1        & CN 102596317 A<br>& KR 10-2012-0095917 A | 1-4 |
| A | WO 2006/107388 A1  (BOSTON SCIENTIFIC SCIMED,<br>INC.),<br>12 October 2006 (12.10.2006),<br>entire text; all drawings<br>& US 2006/0224034 A1 | 1-4 |
| A | WO 2013/125720 A1  (Toppan Printing Co., Ltd.),<br>29 August 2013 (29.08.2013),<br>entire text; all drawings<br>& JP 2013-174458 A | 1-4 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 13 February 2015 (13.02.15) | 24 February 2015 (24.02.15) |

| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |
|---|---|

Form PCT/ISA/210 (second sheet) (July 2009)

**EP 3 095 483 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011055670 A